# EUROPÄISCHE PATENTSCHRIFT

(11) **EP 1 267 846 B1**
(45) Veröffentlichungstag und Bekanntmachung des Hinweises auf die Patenterteilung: **23.11.2005**
(21) Anmeldenummer: 01936095.7
(22) Anmeldetag: 17.03.2001
(51) Int. Cl.: A61K 9/70, A61K 31/496, A61P 1/08

(54) **TRANSDERMALES THERAPEUTISCHES SYSTEM ZUR ABGABE VON LERISETRON**
TRANSDERMAL THERAPEUTIC SYSTEM FOR THE DELIVERY OF LERISETRON
SYSTEME THERAPEUTIQUE TRANSDERMIQUE POUR ADMINISTRER DU LERISETRON

(30) Priorität: 30.03.2000 DE 10015783
(43) Veröffentlichungstag der Anmeldung: 02.01.2003
(73) Patentinhaber: LTS LOHMANN Therapie-Systeme AG, 56626 Andernach (DE)
(72) Erfinder: SELZER, Torsten, 56564 Neuwied (DE); BOTZEM, Petra, 56626 Andernach (DE); HOFFMANN, Gerd, 56566 Neuwied (DE); KINDEL, Heinz, 56581 Ehlscheid (DE)
(74) Vertreter: Flaccus, Rolf-Dieter
(86) Internationale Anmeldenummer: PCT/EP2001/003089
(87) Internationale Veröffentlichungsnummer: WO 2001/074338

(56) Entgegenhaltungen:
- WO-A-00/27371
- WO-A-99/17755
- US-A- 5 256 665

## Beschreibung

Die vorliegende Erfindung betrifft pharmazeutische zubereitungen zur Verabreichung des Wirkstoffs Lerisetron an die Haut. Sie betrifft ferner die Verwendung solcher Zubereitungen zur transdermalen Applikation dieses Wirkstoffs an Patienten zur Prävention und Therapie von Übelkeit und Erbrechen.

Der Wirkstoff Lerisetron gehört zur Klasse der selektiven 5-HT3-Rezeptor-Antagonisten. Er eignet sich allgemein zur Behandlung von Übelkeit und Erbrechen. Insbesondere kann dieser Wirkstoff eingesetzt werden, um die durch Strahlen- oder Chemotherapie verursachte Übelkeit oder das Erbrechen zu unterdrücken oder zu verhindern.

In US 5,256,665 wird ein Verfahren zur Herstellung von 2-Piperazinylbenzimidazol-Derivaten, u. a. des wirkstoffs Lerisetron, beschrieben.

Bei der oralen Verabreichung dieses Wirkstoffs können - insbesondere während einer Chemo- oder Strahlentherapie - Probleme auftreten, wie z. B. gastrointestinale Intoleranz, niedrige enterale Absorption und schnelle "First-Pass"-Metabolisierung in der Leber. Vor allem der letztgenannte Effekt kann eine erhöhte Applikationsfrequenz erforderlich machen.

Eine Umgehung des Gastrointestinaltraktes und damit auch des "First-Pass"-Effekts läßt sich grundsätzlich durch eine transdermale Verabreichung des jeweiligen Wirkstoffes erreichen, beispielsweise durch Verwendung von transdermalen therapeutischen Systemen (TTS). Dabei handelt es sich um Darreichungsformen, die auf die Haut appliziert werden und den enthaltenen Wirkstoff an die Haut abgeben. Allgemein können TTS den therapeutischen Wert einer Arzneistoffzubereitung erhöhen, indem sie eine konstante Abgabe des Arzneistoffes über einen verlängerten Zeitraum in das Blutkompartiment gewährleisten.

Derartige TTS weisen typischerweise einen Aufbau aus einer arzneistoffundurchlässigen Trägerschicht, einer arzneistoffhaltigen Reservoirschicht, gegebenenfalls einer Steuermembran, sowie einer Haftkleberschicht zur Befestigung auf der Haut auf, wobei letztere mit der arzneistoffhaltigen Reservoirschicht identisch sein kann. Die arzneistoffhaltige Schicht kann noch weitere inhaltsstoffe enthalten, z. B. Weichmacher, Klebrigmacher, Lösungsvermittler, Stabilisatoren, Füllstoffe, Trägerstoffe und Permeationsbeschleuniger. Die hierfür in Frage kommenden pharmazeutisch unbedenklichen Substanzen sind dem Fachmann grundsätzlich bekannt.

Auch wenn TTS als Darreichungsformen grundsätzlich bekannt sind, stellt die Formulierung eines bestimmten Wirkstoffs, z. B. Lerisetron, als TTS eine gewisse Herausforderung dar, und es können verschiedene Probleme auftreten. So muß ein TTS, um therapeutisch eingesetzt werden zu können, einen genügend hohen Wirkstoff-Flux durch die Haut ermöglichen. Es muß ferner eine gute Stabilität aufweisen und darf insbesondere während der Lagerung keinen Veränderungen unterliegen. Die Auswahl geeigneter Polymere für das Wirkstoff-reservoir kann sich als schwierig erweisen, da diese Polymere kompatibel sein müssen zu dem jeweiligen Wirkstoff. Außerdem muß das TTS kostengünstig hergestellt werden können.

Die Aufgabe der vorliegenden Erfindung war es deshalb, eine transdermale Darreichungsform für den Wirkstoff Lerisetron bereitzustellen, welche einen genügend hohen Wirkstoff-Flux in vivo ermöglicht, und welche mittels gängiger Herstellungsverfahren kostengünstig produziert werden kann.

Erfindungsgemäß wird diese Aufgabe gelöst durch eine Lerisetron enthaltende pharmazeutische Zubereitung in Form eines transdermalen therapeutischen Systems (TTS) gemäß Anspruch 1, welches ein Lerisetron enthaltendes, mindestens einschichtiges haftklebendes Wirkstoffreservoir auf der Basis von Silikonhaftkleber(n) aufweist. Diese Haftkleber, und gegebenenfalls weitere Polymere, bilden die Polymermatrix des Wirkstoffreservoirs. Als Silikonhaftkleber werden Haftkleber basierend auf einer Polydimethylsiloxanstruktur verstanden.
Das Wirkstoffreservoir ist mit einer wirkstoffundurchlässigen Schutzschicht verbunden. Ferner weist die erfindungsgemäße pharmazeutische Zubereitung in Form eines TTS eine ablösbare Schutzschicht auf, welche vor der Applikation des haftklebenden TTS auf die Haut von der Klebeschicht entfernt wird.

Es hat sich gezeigt, daß die oben erwähnte Formulierung auf der Basis von Silikonhaftkleber(n) besonders vorteilhaft ist, da Lerisetron einerseits eine hohe Permeabilität in der Silikonmatrix des Wirkstoffreservoirs, andererseits aber eine niedrige Affinität zu dieser Matrix besitzt. Zudem weisen Silikonhaftkleber eine hohe Kompatibilität zu dem Wirkstoff Lerisetron auf. Weiterhin ist von Vorteil, daß die Klebeeigenschaften auch bei Temperatur- und Feuchtigkeitsschwankungen konstant bleiben.

Erfindungsgemäß können Lerisetron enthaltende pharmazeutische Zubereitungen in Form von transdermalen therapeutischen Systemen gemäß Anspruch 2 auch ein mindestens einschichtiges haftklebendes Wirkstoffreservoir aufweisen, welches auf der Basis von Polymeren aufgebaut ist, die aus der Polyisobutylene, Polyterpene, Ethylenvinylacetat-Copolymere, Synthesekautschuke und Heißschmelzkleber umfassenden Gruppe ausgewählt sind.
Ferner kann das Lerisetron enthaltende Wirkstoffreservoir der erfindungsgemäßen TTS auch aus einer Mischung von mindestens zwei Polymeren aufgebaut sein, wobei diese Polymere aus der Gruppe ausgewählt werden können, welche Silikonhaftkleber, Polyisobutylene, Polyterpene, Ethylenvinylacetat-Copolymere, Synthesekautschuke und Heißschmelzkleber umfaßt.

Grundsätzlich ist das Wirkstoffreservoir aus einer mindestens einschichtigen Polymermatrix aufgebaut, welche den Wirkstoff Lerisetron und ggf. die weiter unten genannten zusätzlichen Inhaltsstoffe enthält.
Gemäß einer besonderen Ausführungsform ist vorgesehen, daß mindestens eine Polymermatrixschicht des Wirkstoffreservoirs Polymerbestandteile aus der Gruppe der substituierten Cellulosen, vorzugsweise aus der Gruppe der Methyl- oder Ethylcellulosen, enthält.

Grundsätzlich kann der Wirkstoff Lerisetron im Wirkstoffreservoir molekular-dispers oder in Lösung vorliegen; allerdings ist auch eine Formulierung möglich, bei welcher der Wirkstoff grob-dispers, kolloidal oder als Suspension vorliegt.
Vorzugsweise wird eine möglichst hohe Wirkstoffkonzentration in der bzw. den wirkstoffhaltigen Schichten des Wirkstoffreservoirs angestrebt, um eine hohe Freisetzungsrate (Wirkstoff-Flux) zu erzielen. Nach Möglichkeit sollte die Konzentration von Leriseton die Sättigungslöslichkeit erreichen; die wirkstoffhaltigen Schichten können auch wirkstoffübersättigt sein, wobei die Sättigungslöslichkeit überschritten wird. Allerdings ist dabei zu beachten, daß bei zu hohen Wirkstoffkonzentrationen die physikalische Stabilität des Wirkstoffs im Wirkstoffreservoir beeinträchtigt werden kann. Bei den erfindungsgemäßen TTS werden deshalb Wirkstoffkonzentrationen im Bereich von 0,1 bis 30 Gew.-% angestrebt, wobei Wirkstoffkonzentrationen im Bereich zwischen 1 und 10 Gew.-% besonders bevorzugt werden; die Konzentrationsangaben beziehen sich auf die Gesamtmasse der wirkstoffhaltigen Schichten.

Wenn der Wirkstoff im Wirkstoffreservoir in gelöster Form vorliegen soll, ist es vorteilhaft, wenn die Formulierung der Polymermatrix des Wirkstoffreservoirs einen Lösungsvermittler enthält. Als Beispiele für solche Lösungsvermittler seien genannt: 1,2-Propandiol, Tetrahydrofurfurylalkohol, Transcutol, Butandiol, Glycerin, PEG 400, Diethyltoluamid, Monoisopropylidenglycerin, wobei 1,2-Propandiol als Lösungsvermittler besonders bevorzugt wird. Als vorteilhaft hat sich herausgestellt, wenn der Anteil des Lösungsvermittlers, bezogen auf das gesamte TTS, zwischen 1 und 50 Gew.-%, vorzugsweise zwischen 5 und 35 Gew.-% beträgt.

Unter Umständen kann durch die Einarbeitung eines Lösungsvermittlers in die Polymermatrix ein Zwei-Phasen-System entstehen, und/oder der Wirkstoff Lerisetron kann als Dispersion vorliegen. Insbesondere in diesen Fällen ist es vorteilhaft, der wirkstoffhaltigen Polymermatrix einen Emulgator beizumischen.
Dabei wird der Emulgator üblicherweise vor der Bildung des Zwei-Phasen-Systems eingearbeitet. Der Emulgator wird entweder in die kohärente äußere Phase gegeben und die disperse Phase wird nach und nach eingearbeitet, oder der Emulgator wird in die disperse innere Phase eingearbeitet. Alternativ kann der Emulgator auch erst nach erfolgter Bildung des Zwei-Phasen-Systems eingearbeitet werden.

Als Emulgatoren kommen beispielsweise Natriumdodecylsulfat, Lecithin, Cetylalkohol, Cetylstearylalkohol, Sorbitanfettsäureester, Polyoxyethylen-Sorbitanfettsäureester, Polyoxyethylen-Fettsäureglyceride und Polyoxyethylenfettsäureester in Frage.

Um während der Applikation der erfindungsgemäßen Lerisetron enthaltenden TTS einen möglichst hohen Wirkstoff-Flux durch die Haut zu erreichen, hat es sich ferner als besonders günstig erwiesen, wenn das wirkstoffhaltige Reservoir zusätzlich mindestens einen Haut-Penetrationsverstärker ("Enhancer") enthält. Als Haut-Penetrationsverstärker eignen sich beispielsweise Stoffe, die ausgewählt sind aus der Gruppe, die Polyoxyethylenfettsäureester, Polyoxyethylen-sorbitanfettsäureester, Sorbitanfettsäureester, Fettsäuren, Fettalkohole, Ester von Fettsäuren mit Methanol, Ethanol oder Isopropanol, und Ester von Fettalkoholen mit Essigsäure oder Milchsäure umfaßt. Beispiele für Penetrationsverstärker sind Decanol, Dodecanol, Ölsäure, Ölsäurediethanolamin, Myristinsäure, Sorbitanmonolaurat, Polyoxylaurylether (z. B. Brij®). Bevorzugt werden Polyoxyethylenfottalkoholether, besonders bevorzugt Polyoxylaurylether (wie Brij®, z. B. Brij® 30) als Hautpenetrationsverstärker eingesetzt. Um den Wirkstoff-Flux zu optimieren, können erfindungsgemäß auch Kombinationen zweier oder mehrerer penetrationsverstärkender Substanzen eingesetzt werden.

Die Polymer-Matrixschicht(en) des Wirkstoffreservoirs kann bzw. können auch Weichmacher enthalten, um die physikalischen Eigenschaften der Haftklebematrix zu beeinflussen. Als Weichmacher eignen sich vor allem Stoffe, die ausgewählt sind aus der Gruppe, welche Kohlenwasserstoffe, Alkohole, Carbonsäuren und ihre Derivate, Ether, Ester und Amine umfaßt. Die Konzentration des/der Weichmacher(s), bezogen auf das Wirkstoffreservoir, kann zwischen 0 bis 30 Gew.-% betragen, vorzugsweise beträgt sie 5 bis 20 Gew.-%.

Das Wirkstoffreservoir der erfindungsgemäßen Lerisetron enthaltenden pharmazeutischen Zubereitungen in Form von TTS ist in der einfachsten Ausführungsform eine einschichtige Polymermatrix. Allerdings sind nach der Erfindung auch weitere Ausführungsformen vorgesehen, welche sich dadurch auszeichnen, daß das TTS einen schichtförmigen Aufbau des Wirkstoffreservoirs mit mindestens zwei Polymer-Matrixschichten aufweist.
Bei einem solchen mehrschichtigen Wirkstoffreservoir kann von der Möglichkeit Gebrauch gemacht werden, daß die einzelnen Polymer-Matrixschichten unterschiedliche Konzentrationen von Wirkstoff, Hautpenetrationsverstärker(n) oder Emulgator(en) aufweisen.
Bei der Verwendung einer mehrschichtigen Polymer-Matrix ergeben sich auch zusätzliche Variationsmöglichkeiten hinsichtlich der Auswahl der Haftkleber-Polymere. Beispielsweise kann es von vorteil sein, wenn mindestens zwei Polymer-Matrixschichten sich hinsichtlich der an ihrem Aufbau beteiligten Polymere unterscheiden, wobei vorzugsweise mindestens eine Polymer-Matrixschicht Polymerbestandteile aus der in Anspruch 2 genannten Gruppe von Polymeren enthält. Auch können die einzelnen Schichten unterschiedliche Silikonhaftkleber aufweisen.

Falls eine Steuerung der Wirkstoff-Freisetzung erforderlich ist und nicht durch andere Mechanismen bewirkt wird, kann das Wirkstoff-Reservoir auf der Abgabeseite (hautnahe Seite) auch mit einer Steuermembran versehen werden, welche die Abgabe des Wirkstoffs an die Haut steuert. Für diesen Zweck geeignete Membran-Materialien sind dem Fachmann bekannt.

Gemäß einer besonderen Ausführungsform kann der Wirkstoff Lerisetron in der erfindungsgemäßen Zubereitung in Form eines TTS auch in einem beutelförmigen Wirkstoffreservoir vorliegen. Dieses beutelförmige Reservoir ist mit einer flüssigen, hochviskosen, halbfesten oder thixotropen wirkstoffhaltigen Matrix gefüllt, wobei es besonders vorteilhaft ist, wenn das halbfeste oder thixotrope Wirkstoffreservoir einen Gelbildner enthält. Dabei muß die der Haut abgewandte Beutelrückseite wirkstoffundurchlässig, die der Haut zugewandte Seite wirkstoffdurchlässig sein. Optional kann auch eine wirkstoffdurchlässige Membran die Steuerung der Wirkstofffreisetzung kontrollieren.

Der Aufbau der erfindungsgemäßen TTS umfaßt neben dem oben besprochenen Wirkstoffreservoir eine wirkstoffundurchlässige Rückschicht sowie eine ebenfalls wirkstoffundurchlässige ablösbare Schutzfolie.
Als Material für die Rückschicht eignen sich eine Vielzahl von hautverträglichen Kunststoffolien, wie z. B. Folien aus Polyvinylchlorid, Ethylenvinylacetat, Vinylacetat, Polyethylen, Polypropylen oder Cellulosederivaten. Besonders geeignet als Material für die Rückschicht sind Polyester, die sich durch besondere Festigkeit auszeichnen. Ferner kann es im Einzelfall nützlich sein, die aus Folienmaterial bestehende Rückschicht mit einer zusätzlichen Auflage zu versehen, z. B. durch Bedampfung mit Metallen oder anderen diffusionssperrenden Zusatzstoffen wie Siliciumdioxid, Aluminiumoxid oder ähnlicher, dem Fachmann bekannter Stoffe.

Für die ablösbare Schutzschicht können grundsätzlich dieselben Materialien verwendet werden wie für die Rückschicht, vorausgesetzt, daß diese Schicht einer geeigneten Oberflächenbehandlung, z. B. Fluorosilikonisierung, unterzogen wird, so daß sie von der von ihr bedeckten Haftklebeschicht ablösbar ist und vor Applikation des TTS abgezogen werden kann. Außerdem können als ablösbare Schutzschichten auch andere Materialien verwendet werden, wie z. B. Polytetrafluorethylen-behandeltes Papier, Cellophan, Polyvinylchlorid oder ähnliche.

Die erfindungsgemäßen Lerisetron enthaltenden TTS werden anhand eines Herstellungsbeispiels nachfolgend näher erläutert.

### Beispiel

Die erfindungsgemäßen TTS können wie folgt hergestellt werden: Zunächst wird der Wirkstoff Lerisetron sowie ein geeigneter Enhancer (z. B. Brij® 30) in einem Lösungsvermittler (z. B. 1,2 Propandiol) gelöst, wobei die Konzentration von Lerisetron möglichst die Sättigungslöslichkeit erreichen sollte. Gegebenenfalls kann die Lösung auch übersättigt sein. Diese Lösung wird mit einer geeigneten Rühr-apparatur in den Silikonkleber, der ebenfalls in einem Lösungsmittel gelöst ist, gegeben und dispergiert, so daß eine möglichst homogene Flüssig-Flüssig-Dispersion entsteht. Diese Dispersion wird mit einer geeigneten Vorrichtung homogen auf eine Trägerfolie (z. B. Rückschicht) beschichtet. Anschließend wird durch eine kontrollierte Trocknung das Lösungsmittel des Silikonklebers sowie eventuelle Anteile des Lösungsvermittlers entfernt. Das so erhaltene Laminat wird anschließend mit einer weiteren Folie (z. B. Schutzfolie) zukaschiert. Zuletzt werden einzelne TTS einer bestimmten Fläche ausgestanzt und in ein adäquates Packmittel verpackt. Es hat sich gezeigt, daß mit einem derartigen TTS eine für therapeutische Zwecke ausreichende Abgaberate von Lerisetron an die Haut erreicht werden kann. Bei Messungen der Wirkstoffpermeation, die bei 37°C an humaner Epidermis vorgenommen wurden, ergaben sich Wirkstoff-Permeationen von 100-600 µg/cm²d. Dieser Wirkstoffflux ist für therapeutische Anwendungen ausreichend.

Das Beispiel zeigt, daß die erfindungsgemäßen Lerisetron enthaltenden TTS mit Herstellungsverfahren hergestellt werden können, die eine einfache und kostengünstige Produktion ermöglichen.

Die erfindungsgemäßen Lerisetron enthaltenden Zubereitungen in Form von TTS können vorteilhaft dazu verwendet werden, um diesen Wirkstoff auf transdermalem Wege an Patienten zu verabreichen, zum Zwecke der Prävention und Therapie von Übelkeit und Erbrechen, beispielsweise bei Übelkeit und Erbrechen, die bzw. das infolge einer Chemo- oder Strahlentherapie des betreffenden Patienten induziert wird.

Die erfindungsgemäß vorgeschlagene transdermale Verabreichung ist gerade in den vorstehend genannten Situationen besonders vorteilhaft, da sie eine systemische Verabreichung des Wirkstoffs Lerisetron unter Umgehung des Magen-Darm-Traktes ermöglicht. Insbesondere bei an Übelkeit und Erbrechen leidenden Patienten ist eine zuverlässige, sichere und wirksame Verabreichung von Arzneistoffen auf oralem Wege fast nicht möglich. Ferner ist die erfindungsgemäß vorgeschlagene transdermale Verabreichung des Wirkstoffs Lerisetron auch patientenfreundlicher als eine entsprechende orale Verabreichung, da auf diese Weise eine unnötige zusätzliche Belastung des ohnehin geschädigten oder gereizten Magen-Darm-Traktes vermieden werden kann.

Ein an Übelkeit oder Erbrechen, z. B. infolge einer Strahlen- oder Chemotherapie leidender Patient, kann zur Linderung oder Ausschaltung dieser Symptome mit dem Wirkstoff Lerisetron behandelt werden, indem man auf die Haut des betreffenden Patienten ein erfindungsgemäßes Lerisetron enthaltendes TTS aufklebt und diesen Vorgang nötigenfalls in gewissen Zeitabständen wiederholt. Auf diese weise kann ein systemischer Wirkstoffspiegel aufgebaut werden, der therapeutisch wirksam ist.
Die erfindungsgemäßen Lerisetron enthaltenden TTS eignen sich auch insbesondere zur Prävention von Übelkeit bzw. Erbrechen bei einer bevorstehenden Chemo- oder Strahlentherapie. In diesem Fall werden die Lerisetron enthaltenden TTS vorzugsweise schon vor Beginn der Chemo- oder Strahlentherapie auf die Haut des jeweiligen Patienten appliziert.

Erfindungsgemäß kann der Wirkstoff Lerisetron folglich verwendet werden, um eine Arzneizubereitung in Form eines TTS zur transdermalen Verabreichung des Wirkstoffs Lerisetron an den Menschen herzustellen, wobei diese Zubereitung zur Prävention und Therapie von Übelkeit oder Erbrechen, vorzugsweise zur Prävention und Therapie von durch Chemotherapie oder Strahlentherapie induzierter Übelkeit oder induziertem Erbrechen geeignet ist.

## Patentansprüche

1. Lerisetron enthaltende pharmazeutische Zubereitung in Form eines transdermalen therapeutischen Systems (TTS), welches eine Rückschicht, ein damit verbundenes, Lerisetron enthaltendes, mindestens einschichtiges haftklebendes Wirkstoffreservoir auf der Basis von Silikonhaftkleber(n), und eine ablösbare Schutzschicht umfaßt.

2. Lerisetron enthaltende pharmazeutische Zubereitung in Form eines transdermalen therapeutischen Systems, welches eine Rückschicht, ein damit verbundenes, Lerisetron enthaltendes, mindestens einschichtiges haftklebendes Wirkstoff-reservoir auf der Basis von Polymeren aus der Polyisobutylene, Polyterpene, Ethylenvinylacetat-Copolymere, Synthesekautschuke und Heißschmelzkleber umfassenden Gruppe, und eine ablösbare Schutzschicht umfaßt.

3. Zubereitung nach Anspruch 1 oder 2, **dadurch gekennzeichnet, daß** das Wirkstoffreservoir eine Kombination von mindestens zwei Haftklebern enthält, ausgewählt aus der Gruppe der in Anspruch 1 und Anspruch 2 genannten Haftkleber.

4. Zubereitung nach einem der vorangehenden Ansprüche, **dadurch gekennzeichnet, daß** mindestens eine Matrixschicht des Wirkstoffreservoirs Polymerbestandteile aus der Gruppe der substituierten Cellulosen, vorzugsweise aus der Gruppe der Methyl- oder Ethylcellulosen, enthält.

5. Zubereitung nach einem der vorangehenden Ansprüche, **dadurch gekennzeichnet, daß** die Wirkstoffkonzentration, bezogen auf die Gesamtmasse der wirkstoffhaltigen Schicht(en), im Bereich von 0,1 bis 30 Gew.-%, vorzugsweise im Bereich zwischen 1 und 10 Gew.-% liegt, wobei besonders bevorzugt ist, wenn die Wirkstoffkonzentration die Sättigungslöslichkeit erreicht, oder diese überschreitet.

6. Zubereitung nach einem der vorangehenden Ansprüche, **dadurch gekennzeichnet, daß** der Wirkstoff Lerisetron molekular-dispers im Wirkstoffreservoir vorliegt.

7. Zubereitung nach einem der Ansprüche 1 bis 5, **dadurch gekennzeichnet, daß** der Wirkstoff Lerisetron grob-dispers, kolloidal oder als Suspension im Wirkstoffreservoir vorliegt.

8. Zubereitung nach einem oder mehreren der vorangehenden Ansprüche, **dadurch gekennzeichnet, daß** das Wirkstoffreservoir als weiteren Bestandteil mindestens einen Lösungsvermittler aus der Gruppe der mehrwertigen Alkohole, vorzugsweise 1,2-Propandiol, enthält.

9. Zubereitung nach einem oder mehreren der vorangehenden Ansprüche, **dadurch gekennzeichnet, daß** das Wirkstoffreservoir als weiteren Bestandteil mindestens einen Hautpenetrationsverstärker, bevorzugt aus der Gruppe der Polyoxyethylenfettalkoholether, besonders bevorzugt Polyoxylaurylether, enthält.

10. Zubereitung nach Anspruch 9, **dadurch gekennzeichnet, daß** der/die Hautpenetrationsverstärker ausgewählt ist/sind aus der Gruppe, die Polyoxyethylenfettsäureester, Polyoxyethylensorbitanfettsäureester, Sorbitanfettsäureester, Fettsäuren, Fettalkohole, Ester von Fettsäuren mit Methanol, Ethanol oder Isopropanol, und Ester von Fettalkoholen mit Essigsäure oder Milchsäure umfaßt.

11. Zubereitung nach Anspruch 9 oder 10, **dadurch gekennzeichnet, daß** das Wirkstoffreservoir mindestens zwei Haut-penetrationsverstärker in Kombination enthält.

12. Zubereitung nach einem oder mehreren der vorangehenden Ansprüche, **dadurch gekennzeichnet, daß** das Wirkstoffreservoir als weiteren Bestandteil mindestens einen Emulgator enthält, vorzugsweise aus der Natriumdodecylsulfat, Lecithin, Cetylalkohol, Cetylstearylalkohol, Sorbitanfettsäureester, Polyoxyethylen-Sorbitanfettsäureester, Polyoxyethylen-Fettsäureglyceride und Polyoxyethylenfettsäureester umfassenden Gruppe.

13. Zubereitung nach einem oder mehreren der vorangehenden Ansprüche, **dadurch gekennzeichnet, daß** das Wirkstoffreservoir als weiteren Bestandteil mindestens einen Weichmacher enthält, wobei die Konzentration des/der Weichmacher(s), bezogen auf das Wirkstoffreservoir, 0 bis 30 Gew.-%, vorzugsweise 5 bis 20 Gew.-% beträgt, und wobei der/die Weichmacher vorzugsweise ausgewählt ist/sind aus der Gruppe, welche Kohlenwasserstoffe, Alkohole, Carbonsäuren und ihre Derivate, Ether, Ester und Amine umfaßt.

14. Zubereitung nach einem oder mehreren der vorangehenden Ansprüche, **dadurch gekennzeichnet, daß** das transdermale therapeutische System einen schichtförmigen Aufbau des Wirkstoffreservoirs mit mindestens zwei Polymer-Matrixschichten aufweist.

15. Zubereitung nach Anspruch 14, **dadurch gekennzeichnet, daß** die mindestens zwei Polymer-Matrixschichten unterschiedliche Konzentrationen von Wirkstoff, Hautpenetrationsverstärker(n) oder Emulgator(en) aufweisen.

16. Zubereitung nach Anspruch 14 oder 15, **dadurch gekennzeichnet, daß** die mindestens zwei Polymer-Matrixschichten sich hinsichtlich der an ihrem Aufbau beteiligten Polymere unterscheiden, wobei vorzugsweise mindestens eine Polymer-Matrixschicht Polymerbestandteile aus der in Anspruch 2 genannten Gruppe von Polymeren enthält.

17. Zubereitung nach einem oder mehreren der vorangehenden Ansprüche, **dadurch gekennzeichnet, daß** das Wirkstoffreservoir des TTS beutelförmig ist und mit einer flüssigen, hochviskosen, halbfesten oder thixotropen wirkstoffhaltigen Matrix gefüllt ist, wobei das halbfeste oder thixotrope Wirkstoffreservoir vorzugsweise einen Gelbildner enthält.

18. Verwendung des Wirkstoffs Lerisetron zur Herstellung einer Arzneizubereitung nach einem der Ansprüche 1 bis 17 zur transdermalen Verabreichung des Wirkstoffs Lerisetron an den Menschen zur Prävention und Therapie von Übelkeit oder Erbrechen, vorzugsweise zur Prävention und Therapie von durch Chemotherapie oder Strahlentherapie induzierter Übelkeit oder induziertem Erbrechen.

## Claims

1. Lerisetron pharmaceutical preparation in the form of a transdermal therapeutic system (TTS) which comprises a backing layer, connected to it an at least single-layer, pressure-sensitively adhesive, lerisetron active substance reservoir based on silicone pressure-sensitive adhesive(s), and a removable protective layer.

2. Lerisetron pharmaceutical preparation in the form of a transdermal therapeutic system which comprises a backing layer, connected to it an at least single-layer pressure-sensitively adhesive lerisetron active substance reservoir based on polymers from the group comprising polyisobutylenes, polyterpenes, ethylenevinyl acetate copolymers, synthetic rubbers and hotmelt adhesives, and a removable protective layer.

3. Preparation according to Claim 1 or 2, **characterized in that** the active substance reservoir comprises a combination of at least two pressure-sensitive adhesives selected from the group of pressure-sensitive adhesives specified in Claim 1 and Claim 2.

4. Preparation according to one of the preceding claims, **characterized in that** at least one matrix layer of the active substance reservoir comprises polymer constituents from the group of the substituted celluloses, preferably from the group of the methyl celluloses or ethyl celluloses.

5. Preparation according to one of the preceding claims, **characterized in that** the active substance concentration, based on the overall mass of the active substance layer(s), is in the range from 0.1 to 30% by weight, preferably in the range between 1 and 10% by weight, it being particularly preferred for the active substance concentration to match or exceed the saturation solubility.

6. Preparation according to one of the preceding claims, **characterized in that** the active substance lerisetron is present in molecularly disperse form in the active substance reservoir.

7. Preparation according to one of Claims 1 to 5, **characterized in that** the active substance lerisetron is present in coarsely disperse form, in colloidal form, or as a suspension in the active substance reservoir.

8. Preparation according to one or more of the preceding claims, **characterized in that** the active substance reservoir comprises as a further constituent at least one solubilizer from the group of the polyhydric alcohols, preferably 1,2-propanediol.

9. Preparation according to one or more of the preceding claims, **characterized in that** the active substance reservoir comprises as a further constituent at least one skin penetration enhancer, preferably from the group of the polyoxyethylene-fatty alcohol ethers, with particular preference polyoxylauryl ethers.

10. Preparation according to Claim 9, **characterized in that** the skin penetration enhancer(s) is (are) selected from the group comprising polyoxyethylene-fatty acid esters, polyoxyethylene-sorbitan fatty acid esters, sorbitan fatty acid esters, fatty acids, fatty alcohols, esters of fatty acids with methanol, ethanol or isopropanol, and esters of fatty alcohols with acetic acid or lactic acid.

11. Preparation according to Claim 9 or 10, **characterized in that** the active substance reservoir comprises at least two skin penetration enhancers in combination.

12. Preparation according to one or more of the preceding claims, **characterized in that** the active substance reservoir comprises as a further constituent at least one emulsifier, preferably from the group comprising sodium dodecyl sulphate, lecithin, cetyl alcohol, cetylstearyl alcohol, sorbitan-fatty acid esters, polyoxyethylene-sorbitan fatty acid esters, polyoxyethylene-fatty acid glycerides and polyoxyethylene-fatty acid esters.

13. Preparation according to one or more of the preceding claims, **characterized in that** the active substance reservoir comprises as a further constituent at least one plasticizer, the concentration of the plasticizer(s), based on the active substance reservoir, being from 0 to 30% by weight, preferably from 5 to 20% by weight, and the plasticizer(s) preferably being selected from the group comprising hydrocarbons, alcohols, carboxylic acids and their derivatives, ethers, esters and amines.

14. Preparation according to one or more of the preceding claims, **characterized in that** the transdermal therapeutic system has a layer-form construction of the active substance reservoir, comprising at least two polymer matrix layers.

15. Preparation according to Claim 14, **characterized in that** the at least two polymer matrix layers have different concentrations of active substance, skin penetration enhancer(s) or emulsifier(s).

16. Preparation according to Claim 14 or 15, **characterized in that** the at least two polymer matrix layers differ in respect of the polymers involved in their construction, at least one polymer matrix layer preferably comprising polymer constituents from the group of polymers specified in Claim 2.

17. Preparation according to one or more of the preceding claims, **characterized in that** the active substance reservoir of the TTS is pouchlike and is filled with a liquid, highly viscous, semisolid or thixotropic active substance matrix, the semisolid or thixotropic active substance reservoir preferably comprising a gel former.

18. Use of the active substance lerisetron for producing a pharmaceutical preparation according to one of Claims 1 to 17 for transdermal administration of the active substance lerisetron to human beings for the prevention and therapy of nausea or vomiting, preferably for the prevention and therapy of vomiting or nausea induced by chemotherapy or radiation therapy.

## Revendications

1. Préparation pharmaceutique contenant du lerisetron sous la forme d'un système thérapeutique transdermique (STT), qui comprend une couche dorsale, un réservoir de substance active adhérant par contact, à base d'un ou plusieurs adhésifs de silicone, constitué d'au moins une couche, et contenant du lerisetron, qui y est fixé, et une couche protectrice détachable.

2. Préparation pharmaceutique contenant du lerisetron sous la forme d'un système thérapeutique transdermique, qui comprend une couche dorsale un réservoir de substance active adhérant par contact, à base de polymères issus du groupe comprenant des polyisobutylènes, des polyterpènes, des copolymères de l'éthylène-acétate de vinyle, des caoutchoucs de synthèse et d'adhésif thermofusible, constitué d'au moins une couche et contenant du lerisetron, qui y est fixé, et une couche protectrice détachable.

3. Préparation selon la revendication 1 ou 2, **caractérisée en ce que** le réservoir de substance active contient une combinaison d'au moins deux adhésifs par contact, choisis dans le groupe des adhésifs par contact mentionnés dans la revendication 1 et la revendication 2.

4. Préparation selon l'une des revendications précédentes, **caractérisée en ce qu'**au moins une couche de matrice du réservoir de substance active contient des constituants polymères du groupe des celluloses substituées, de préférence du groupe des méthyl- ou éthyl-celluloses.

5. Préparation selon l'une des revendications précédentes, **caractérisée en ce que** la concentration de substance active, par rapport à la masse totale de la ou des couche(s) contenant la substance active, se situe dans un intervalle allant de 0,1 à 30 % en poids, de préférence dans un intervalle compris entre 1 et 10 % en poids, et l'on préfère en particulier que la concentration de substance active atteigne la solubilité à saturation ou la dépasse.

6. Préparation selon l'une des revendications précédentes, **caractérisée en ce que** la substance active lerisetron se présente sous une forme moléculaire dispersée dans le réservoir de substance active.

7. Préparation selon l'une des revendications 1 à 5, **caractérisée en ce que** la substance active lerisetron se présente sous forme grossièrement dispersée, colloïdale ou sous forme de suspension dans le réservoir de substance active.

8. Préparation selon une ou plusieurs des revendications précédentes, **caractérisée en ce que** le réservoir de substance active contient comme autre constituant au moins un agent de solubilisation du groupe des alcools polyvalents, de préférence le 1,2-propanediol.

9. Préparation selon une ou plusieurs des revendications précédentes, **caractérisée en ce que** le réservoir de substance active contient comme autre constituant au moins un agent de renforcement de la pénétration dans la peau, de préférence du groupe des alcool-éthers gras de polyoxyéthylène, de façon particulièrement préférée l'éther polyoxylaurique.

10. Préparation selon la revendication 9, **caractérisée en ce que** le ou les agents de renforcement de la pénétration dans la peau est ou sont choisi(s) dans le groupe qui comprend les esters d'acides gras de polyoxyéthylène, les esters d'acides gras de polyoxyéthylènesorbitane, les esters d'acides gras de sorbitane, les acides gras, les alcools gras, les esters d'acides gras avec le méthanol, l'éthanol ou l'isopropanol, et les esters d'alcools gras avec l'acide acétique ou l'acide lactique.

11. Préparation selon la revendication 9 ou 10, **caractérisée en ce que** le réservoir de substance active contient au moins deux agents de renforcement de la pénétration dans la peau en combinaison.

12. Préparation selon une ou plusieurs des revendication précédentes, **caractérisée en ce que** le réservoir de substance active contient comme autre constituant au moins un émulsifiant, de préférence du groupe comprenant le dodécylsulfate de sodium, la lécithine, l'alcool cétylique, l'alcool cétylstéarylique, les esters d'acides gras de sorbitane, les esters d'acides gras de polyoxyéthylène-sorbitane, les glycérides d'acides gras de polyoxyéthylène et les esters d'acides gras de polyoxyéthylène.

13. Préparation selon une ou plusieurs des revendications précédentes, **caractérisée en ce que** le réservoir de substance active contient comme autre constituant au moins un plastifiant, la concentration du ou des plastifiant(s), par rapport au réservoir de substance active, s'élevant à 0 à 30 % en poids, de préférence à 5 à 20 % en poids, et le ou les plastifiant(s) étant choisi(s) dans le groupe qui comprend les hydrocarbures, les alcools, les acides carboxyliques et leurs dérivés, éthers, esters et amines.

14. Préparation selon une ou plusieurs des revendications précédentes, **caractérisée en ce que** le système thérapeutique transdermique présente une constitution stratifiée du réservoir de substance active avec au moins deux couches de matrice polymère.

15. Préparation selon la revendication 14, **caractérisée en ce que** les au moins deux couches de matrice polymère présentent différentes concentrations de substance active, d'agent(s) de renforcement de la pénétration dans la peau ou d'émulsifiant(s).

16. Préparation selon la revendication 14 ou 15, **caractérisée en ce que** les au moins deux couches de matrice polymèrese différencient quant aux polymères qui participent à leur constitution, au moins une couche de matrice polymère contenant de préférence des constituants polymères du groupe de polymères mentionné dans la revendication 2.

17. Préparation selon une ou plusieurs des revendications précédentes, **caractérisée en ce que** le réservoir de substance active du STT est en forme de sachet et est rempli d'une matrice liquide hautement visqueuse, semi-solide ou thixotrope contenant une substance active, le réservoir de substance active semi-solide ou thixotrope contenant de préférence un agent gélifiant.

18. Utilisation de la substance active lerisetron pour la fabrication d'une préparation médicamenteuse selon l'une des revendications 1 à 17 en vue de l'administration transdermique de la substance active lerisetron à l'homme pour la prévention et le traitement de la nausée ou des vomissements, de préférence pour la prévention et le traitement de la nausée ou des vomissements induits par la chimiothérapie ou la radiothérapie.
